# EUROPEAN PATENT APPLICATION

(11) **EP 3 851 515 A1**
(43) Date of publication of application: **21.07.2021**
(21) Application number: 19860953.9
(22) Date of filing: 13.09.2019
(51) Int. Cl.: C12M 3/00, C12M 1/00

(54) **CELL CULTURE SUBSTRATE MADE OF NONWOVEN FABRIC MANUFACTURED USING ELECTROSPINNING AND METHOD OF MANUFACTURING THE SAME**

(30) Priority: 14.09.2018 US 201862731443 P; 29.03.2019 JP 2019066448
(71) Applicant: Orthorebirth Co., Ltd., Yokohama-shi, Kanagawa 224-0033 (JP)
(72) Inventor: MAKITA Masashi, Yokohama-shi, Kanagawa 224-0033 (JP); OSAKA Naoya, Yokohama-shi, Kanagawa 224-0033 (JP); TAIRA Hiroyuki, Yokohama-shi, Kanagawa 224-0033 (JP)
(74) Representative: Weihs, Bruno Konrad
(86) International application number: PCT/JP2019/036052
(87) International publication number: WO 2020/054834

(57) **Abstract**

A cell culture substrate used to proliferate mesenchymal stem cells (MSCs) while maintaining differentiation potential is produced and provided on a commercial basis. A nonwoven fabric made of resin fibers spun by using an electrospinning method comprises a plurality of resin fibers having an outer diameter of 10 to 50µm, wherein the plurality of resin fibers entangle each other in random directions, and the plurality of entangled resin fibers adhere and connect with each other at a position where they come into contact to constitute a network structure. The network structure forms a substantially oval-shaped mesh holes having a diameter of 100 to 200µm surrounded by curved resin fibers. Myriad of bubble pores with a diameter of 0.1 to 3µm are formed in the fibers constituting the nonwoven fabric over the entire surface area of the resin fiber.

## Description

### [Technical Field]

Present invention relates to a cell culture substrate used for proliferating mesenchymal stem cells (MSCs) while maintaining their multipotency, and a method for producing the same. More specifically, present invention relates to a nonwoven fabric made of a fiber having a diameter of a micrometer range produced by using electrospinning, and a method of producing a nonwoven fabric made of a fiber having a diameter of a micrometer range, and a cell culture substrate made of a fiber having a diameter of a micrometer range produced by the method.

### [Background Art]

Human and other multicellular organisms are composed of many cells and non-cellular fine components (extracellular matrix) mostly collagen. By binding or adhering to other cell(s) or extracellular matrix, cells sense their own environment and determine their fate (whether they proliferate, suicide, or change into another type of cell). In in vitro cell culture, scaffold materials (cell culture substrates) function as extracellular matrices (ECMs). In order to grow living cells in vitro, it is essential that cell culture substrates function in the same way as ECMs to attach cells in vivo.

Recently, a nonwoven fabric composed of nanofibers spun using an electrospinning method has been proposed as a cell culture substrate. Since a nonwoven fabric composed of fibers spun using an electrospinning method forms a three-dimensional structure approximated to an extracellular matrix (ECM), it can be used as an excellent three-dimensional cell culture substrate that replaces a plate type conventional two-dimensional cell culture (Non-Patent Document 1)

It is necessary that a nonwoven fabric used as a three-dimensional cell culture substrate has a space in which cells enter between the fibers and can supply nutrients and oxygen contained in the medium. Since human MSCs usually have a size of 10µm or more (Non-Patent Document 2), it is desirable that the inter-fiber space is equal to or slightly greater than that in order to grow cells on a substrate.

However, nonwoven fabrics produced by using a conventional electrospinning method have extremely fine fibers densely entangled with each other. Therefore, it is difficult to form a sufficient gap between fibers. When the interstitial space between the fibers is smaller than the size of the cells, entry of the cells is prevented and performance of the nonwoven fabric as a three-dimensional culture substrate is impaired. Therefore, in order to overcome this problem, an attempt has been proposed to form a void between fibers (Non-Patent Document 3). Inter-fiber space can be enlarged by increasing the outer diameter of the fibers. However, because conventional electrospinning method uses bending instability phenomena to convert fibers emitted from the nozzle into ultrafine fibers, it has been difficult to increase the fiber diameter.

On the other hand, if the gap between fibers of the nonwoven fabric is too large, seeded cells pass through the nonwoven fabric in the medium. As a result, they cannot stay in the nonwoven fabric. Therefore, it is necessary that the nonwoven fabric used as a cell culture substrate has a space for allowing cells to enter into the inter-fiber space and proliferate, and at the same time, has a structure for capturing seeded cells and adhering the cells to the substrate fibers in that state.

In order for the nonwoven fabric to function as a pseudo-ECM, it is necessary that the surface of the fiber constituting the nonwoven fabric is covered with a protein. Cells cannot adhere directly to the polymer surface of the fibers because they cannot grow but will die by apoptosis unless they are adherent to the extracellular matrix. When the nonwoven fabric is immersed in the cell culture medium, protein contained in the medium is adsorbed on the surface of the fiber to form a layer of protein, so that it becomes possible for the cells to adhere to the substrate surface via the layer of protein.

Proteins are known to adsorb by hydrophobic interactions to polymer surfaces. Proteins are composed of amino acids with hydrophilic groups and amino acids with hydrophobic groups. Because surface of a protein molecule has a mosaic structure having hydrophilic and hydrophobic portions, the hydrophobic portions tend to adsorb to the surface of a polymer material composed of a variety of molecules in order to avoid contact with water molecules. Since PLGA, PLLA, and PCL have hydrophobic groups, they are suitable resins for obtaining adsorption by hydrophobic interaction with proteins.

Further, adhesion of cells to a substrate is greatly influenced by the topography of the substrate surface. Therefore, it has been proposed that the adhesion of cells is improved by processing the microstructure on the substrate surface using photolithography technology (Non-Patent Document 4). It has been reported that cells preferentially adhere along their structure to a polymer surface having a physically uneven structure

### (Non-Patent Document 5)

### [Prior art documents]

### [Non-patent document]

[Non-Patent Document 1] "Controlling the porosity of fibrous scaffolds by modulating the fiber diameter and packing density" Soliman et al Journal of Biomaterials Research A March 1, 2011 Vol. 96A ISSUE 3.
[Non-Patent Document 2] "3D Scaffold of Electrosprayed Fibers with Large Pore Size for Tissue Regeneration" Hong et al. Acta Biomater Dec 1, 2011
[Non-Patent Document 3] "Enhancing cell infiltration of electrospun fibrous scaffolds in tissue regeneration" Jinglei Wu et al. Bioactive Materials Acta Biomater July 26, 2016.
[Non-Patent Document 4] "Cellular Response by Three-Dimensional Surface Structure of Culture Substrates" Mar. 2017, Journal of the Society of Physical Properties of Ito-shi Japan, Vol. 28, No. 1/2
[Non-Patent Document 5] "Reactions between Polymers and Cells" Raft-artificial Polymers, Vol. 41, October, pp. 702-705 (1992)

### [SUMMARY OF INVENTION]

### [Problem to be solved by the invention]

In order to culture and proliferate a mesenchymal stem cells (MSCs), which is a scaffold-dependent cell, in vitro using a cell culture substrate made of a nonwoven fabric produced by an electrospinning method, it is necessary that a nonwoven fabric used as a cell culture substrate satisfies all requirements regarding an inter-fiber space, a protein adsorption on a substrate surface, a physical topography, and the like. Until now, however, nonwoven fabrics used for cell culture substrates could not be produced and offered inexpensively on a commercial basis to meet all of these requirements.

### [Means for Solving Problems]

In order to solve the above problems, inventors of the present invention have repeatedly conducted experiments and carefully studied and succeeded to stably electrospin fibers with a diameter of 10 to 50µm by setting the condition of electrospinning which the inventors originally developed. Further, when the fibers thus spun were deposited on a rotating drum, it was possible to recover a nonwoven fabric in which the fibers were adherently connected to each other at a portion in contact with each other to form a network structure.

When the surface of the spun fibers was observed in detail, it was discovered that numerous bubble pores having a diameter of about 1µm were formed over the entire area of the fiber surface. The inventors of the present invention found that when a nonwoven fabric made of such a resin fiber is immersed in a cell culture medium, high protein adsorption is obtained on the fiber surface, and good cell adhesion is obtained.

Based on the above findings, inventors of the present invention reached the invention of a cell culture substrate prepared by using a nonwoven fabric made of resin fibers spun by using an electrospinning method.

The nonwoven fabric comprises a plurality of resin fibers having an outer diameter of 10 to 50µm, wherein the plurality of resin fibers are entangled each other in random directions.

A network structure is constituted by connecting a plurality of the entangled resin fibers at a point in contact with each other, wherein the network structure comprises substantially oval-shaped mesh holes having a diameter of about 100 to 200µm surrounded by a curved fiber.

The fibers constituting the nonwoven fabric do not contain an inorganic filler, and a myriad of bubble pores having a diameter of 0.1 to 3µm are formed over the entire surface of the fiber.

Preferably, said nonwoven fabric has a thickness of 0.1mm to 0.4mm.

Preferably, said resin fibers comprise PLGA or PLLA having a molecular weight of 0.2 million to 0.4 million.

Preferably, the resin fiber is made of PCL

### [Advantage of the Invention]

Since the nonwoven fabric produced by the method of one embodiment of the present invention has an outer diameter of 10 to 50µm of the fiber, space between the fibers is large, and cells can easily enter into internal structure of the nonwoven fabric and adhere to the surface of the fiber and proliferate therein.

Since a nonwoven fabric produced by the method of an embodiment of the present invention forms a substantially oval-shaped mesh holes of about 100 to 200µm in diameter surrounded by curved fibers, it is highly capable of capturing seeded cells in the fibrous structure of the nonwoven fabric.

In the fibers constituting the nonwoven fabric produced using the method of an embodiment of the present invention, myriad of bubble pores having a diameter of about 1µm are formed over the entire area of the surface of the fiber, so that the specific surface area is greatly increased and the protein adsorption performance is high. Further, since a physically uneven structure is formed on the surface of the fiber by a myriad of bubble pores, cells captured in the nonwoven fabric can be attached and adhered along the uneven structure.

When MSCs are cultured using a nonwoven fabric produced by the method of an embodiment of the present invention as a cell culture scaffold, highly efficient cell proliferation is achieved. In addition, when the nonwoven fabric in which cells became confluent therein is brought into contact with an unseeded nonwoven fabric, cells are transferred to the unseeded nonwoven fabric, so that "enlarged culture" can be achieved. This may eliminate the need for or reduce the number of passages.

Since the fiber constituting the nonwoven fabric produced by the method of one embodiment of the present invention is made of 100% resin and does not contain inorganic particles such as a calcium compound, when immersed in a medium for growth, inorganic ions such as calcium are not eluted from the fiber, so that differentiation of MSC is not induced and can be proliferated while maintaining the differentiation ability of MSC

Since present invention makes it possible to inexpensively and efficiently produce a nonwoven fabric used as a cell culture scaffold using an electrospinning method, proliferation of MSCs on a commercial basis can be realized.

### [BRIEF DESCRIPTION OF DRAWINGS]

[Figure 1] FIG. 1 illustrates an electrospinning apparatus for use in the method of the present invention.
[Figure 2] Fig. 2(A) shows SEM images (300 times) of sheets produced using Experiment 1 (28kV, 200mm, 18G, 10ml/h) of the present invention. Fig. 2(B) shows SEM images (70 times) of sheets prepared using the same method. Fig. 2(C) shows SEM images (3000 times) of sheets prepared using the same method. 2 (D) shows an appearance photograph of a cell culture substrate made of a sheet produced using the method of the present invention. 2 (E) shows the results of measuring the fiber length of the fibers spun by the method of the present invention.
[Figure 3] Fig. 3(A) shows SEM images (500 times) of sheets produced using Experiment 2 (21kV, 150mm, 18G, 10ml/h) of the present invention. Fig. 3(B) shows SEM images (100 times) of sheets prepared using the same method. Fig. 3(C) shows SEM images (50 times) of sheets prepared using the same method.
[Figure 4] Fig. 4(A) shows SEM images (500 times) of sheets produced using Experiment 3 (14kV, 100mm, 18G, 10ml/h) of the present invention. Fig. 4(B) shows SEM images (100 times) of sheets prepared using the same method. Fig. 4(C) shows SEM images (22 times) of sheets prepared using the same method.

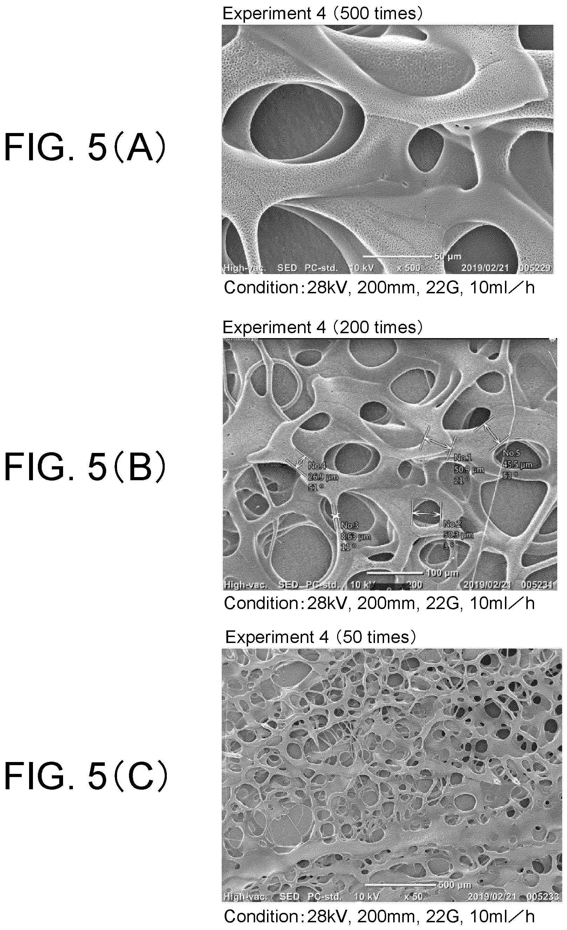

[Figure 5] Fig. 5(A) shows SEM images (500 times) of sheets produced using Experiment 4 (28kV, 200mm, 22G, 10ml/h) of the present invention. Figure 5(B) shows the SEM image (200× magnification) of a nonwoven fabric produced using the same method. Fig. 5(C) shows SEM images (50 times) of sheets prepared using the same method.
[Figure 6] Fig. 6 shows the results of comparative experiment 1.
[Figure 7] Figure 7 shows the results of comparative experiment 2.
[Figure 8] Figure 8 shows the results of comparative experiment 3.

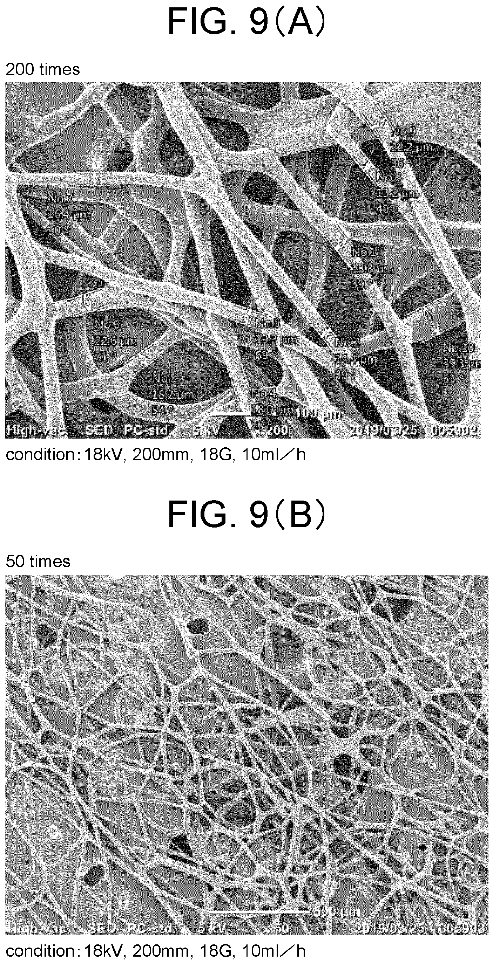

[Figure 9] Fig. 9(A) shows a SEM photograph (200 times) of a sheet fabricated by spinning at an applied voltage of 18kV, a distance of 200mm between the nozzle and the drum, a nozzle aperture 18G, and a spinning solution delivery rate of 10ml/h, using the methods of the present invention. Fig. 9(B) shows a SEM photograph (50 times) of a sheet fabricated by spinning at an applied voltage of 18kV, a distance between the nozzle and the drum of 200mm, a nozzle aperture 18G, and a spinning solution delivery rate of 10ml/h, using the methods of the present invention.

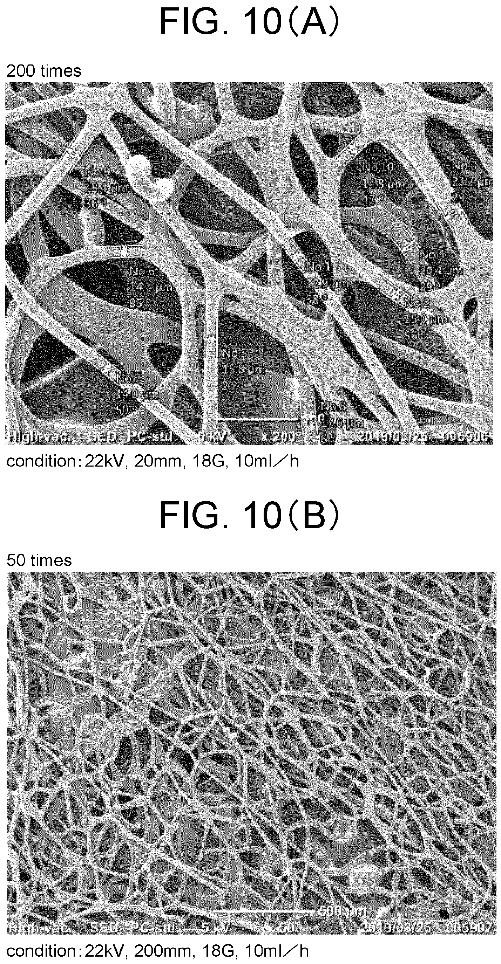

[Figure 10] Fig. 10(A) shows a SEM photograph (200 times) of a sheet fabricated by spinning at an applied voltage of 22kV, a distance between the nozzle and the drum of 200mm, a nozzle aperture 18G, and a spinning solution delivery rate of 10ml/h, using the methods of the present invention. Fig. 10(B) shows a SEM photograph (50 times) of a sheet fabricated by spinning at an applied voltage of 22kV, a distance between the nozzle and the drum of 200mm, a nozzle aperture 18G, and a spinning solution delivery rate of 10ml/h using the methods of the present invention.

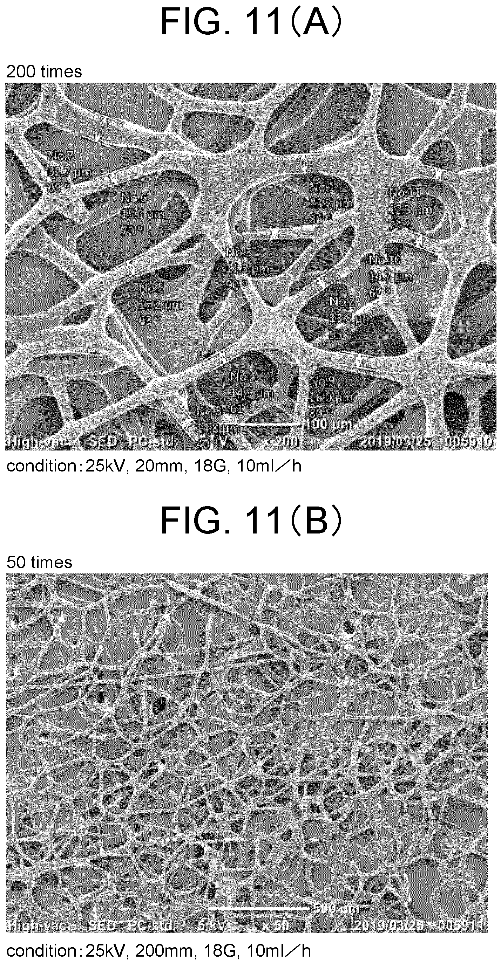

[Figure 11] Fig. 11(A) shows a SEM photograph (200 times) of a sheet fabricated by spinning at an applied voltage of 25kV, a distance between the nozzle and the drum of 200mm, a nozzle aperture 18G, and a spinning solution delivery rate of 10ml/h using the methods of the present invention. Fig. 11(B) shows a SEM photograph (50 times) of a sheet fabricated by spinning at an applied voltage of 25kV, a distance between the nozzle and the drum of 200mm, a nozzle aperture 18G, and a spinning solution delivery rate of 10ml/h using the methods of the present invention.

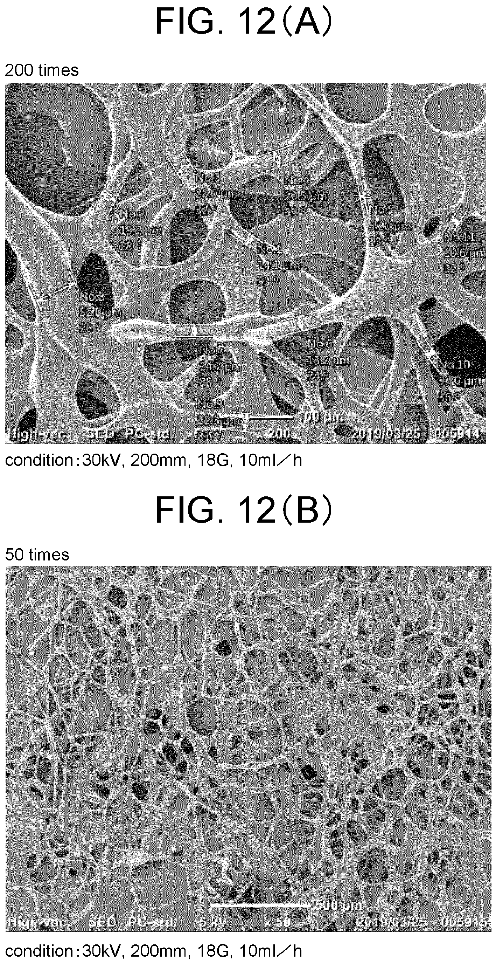

[Figure 12] Fig. 12(A) shows a SEM photograph (200 times) of a sheet fabricated by spinning at an applied voltage of 30kV, a distance of 200mm between the nozzle and the drum, a nozzle aperture 18G, and a spinning solution delivery rate of 10ml/h, using the methods of the present invention. Fig. 12(B) shows a SEM photograph (50 times) of a sheet fabricated by spinning at an applied voltage of 30kV, a distance between the nozzle and the drum of 200mm, a nozzle aperture 18G, and a spinning solution delivery rate of 10ml/h using the methods of the present invention.

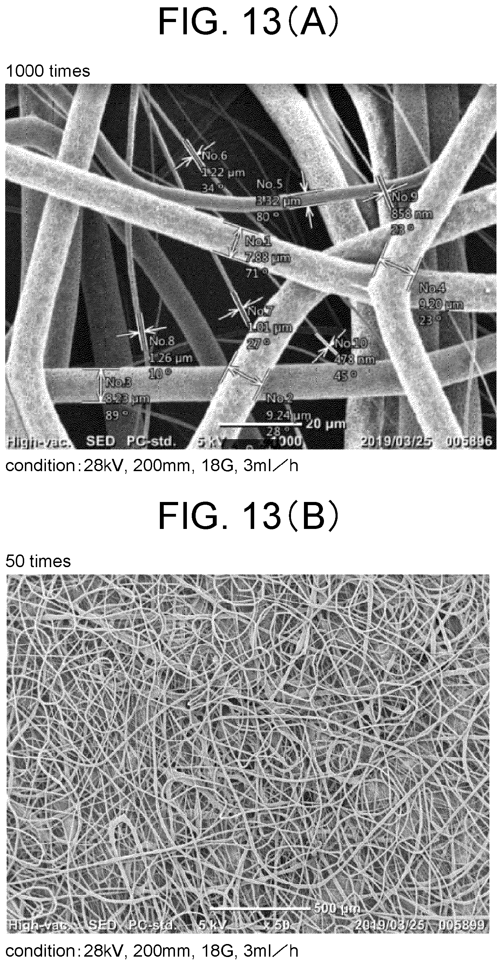

[Figure 13] Fig. 13(A) shows a SEM photograph (1000 times) of a sheet fabricated by spinning at an applied voltage of 28kV, a distance between the nozzle and the drum of 200mm, a nozzle aperture 18G, and a spinning solution delivery rate of 3ml/h using the inventive process. Fig. 13(B) shows a SEM photograph (50 times) of a sheet fabricated by spinning at an applied voltage of 28kV, a distance between the nozzle and the drum of 200mm, a nozzle aperture 18G, and a spinning solution delivery rate of 3ml/h using the methods of the present invention.

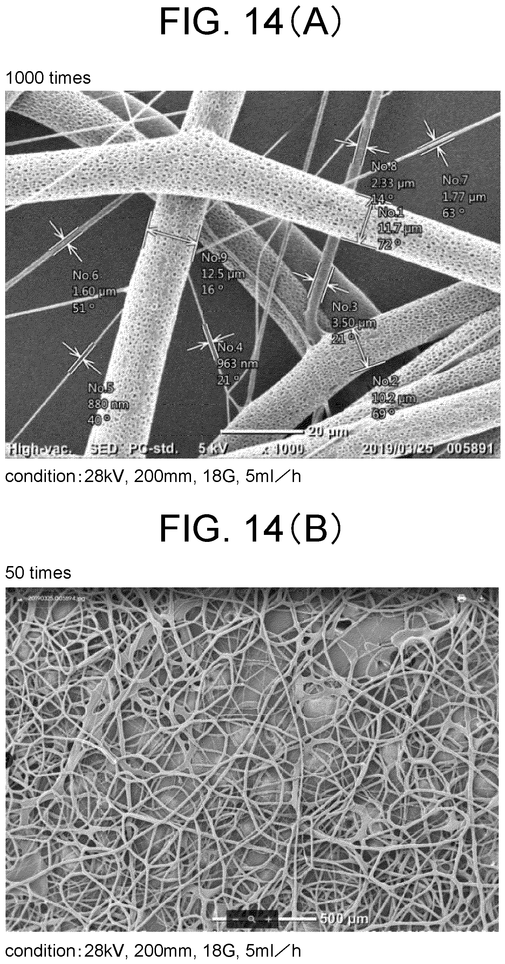

[Figure 14] Fig. 14(A) shows a SEM photograph (1000 times) of a sheet fabricated by spinning at an applied voltage of 28kV, a distance 200mm between the nozzle and the drum, a nozzle aperture 18G, and a spinning solution delivery rate of 5ml/h, using the methods of the present invention. Fig. 14(B) shows a SEM photograph (50 times) of a sheet fabricated by spinning at an applied voltage of 28kV, a distance 200mm between the nozzle and the drum, a nozzle aperture 18G, and a spinning solution delivery rate of 5ml/h, using the methods of the present invention.

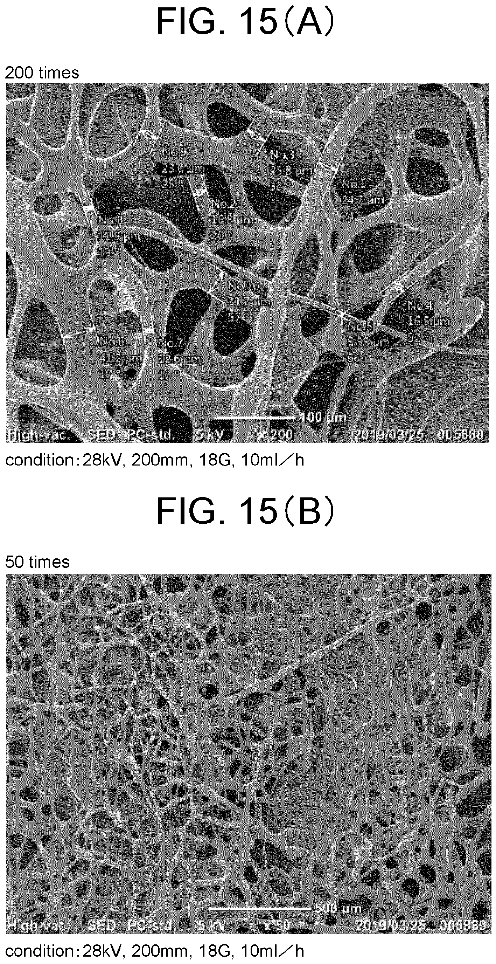

[Figure 15] Fig. 15(A) shows a SEM photograph (1000 times) of a sheet fabricated by spinning at an applied voltage of 28kV, a distance between the nozzle and the drum of 200mm, a nozzle aperture 18G, and a spinning solution delivery rate of 10ml/h using the inventive process. Fig. 15(B) shows a SEM photograph (50 times) of a sheet fabricated by spinning at an applied voltage of 28kV, a distance between the nozzle and the drum of 200mm, a nozzle aperture 18G, and a spinning solution delivery rate of 10ml/h using the methods of the present invention.

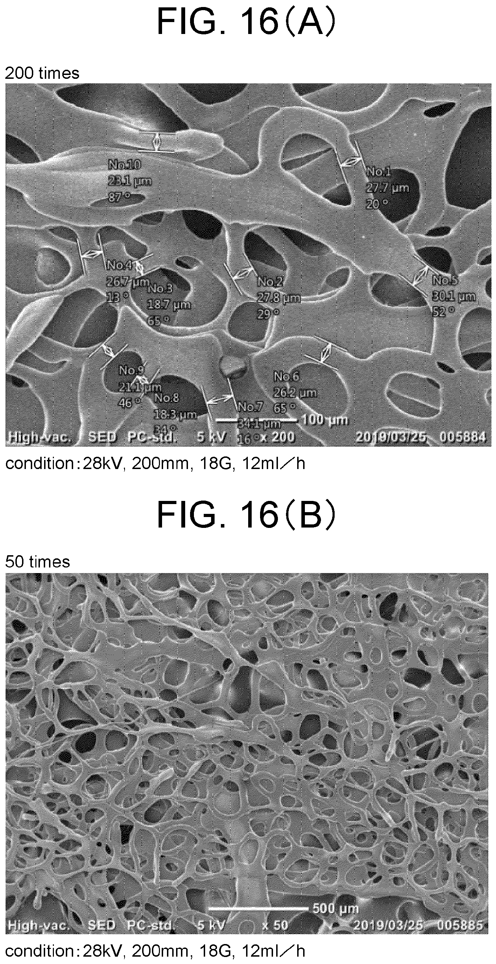

[Figure 16] Fig. 16(A) shows a SEM photograph (200 times) of a sheet fabricated by spinning at an applied voltage of 28kV, a distance between the nozzle and the drum of 200mm, a nozzle aperture 18G, and a spinning solution delivery rate of 12ml/h, using the methods of the present invention. Fig. 16(B) shows a SEM photograph (50 times) of a sheet fabricated by spinning at an applied voltage of 28kV, a distance between the nozzle and the drum of 200mm, a nozzle aperture 18G, and a spinning solution delivery rate of 12ml/h, using the methods of the present invention.

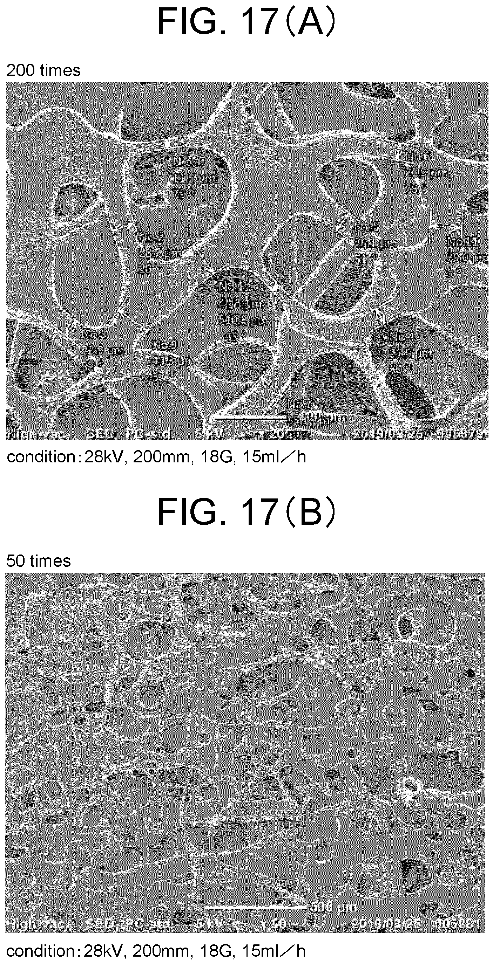

[Figure 17] Fig. 17(A) shows a SEM photograph (1000 times) of a sheet fabricated by spinning at an applied voltage of 28kV, a distance 200mm between the nozzle and the drum, a nozzle aperture 18G, and a spinning solution delivery rate of 15ml/h, using the methods of the present invention. Fig. 17(B) shows a SEM photograph (50 times) of a sheet fabricated by spinning at an applied voltage of 28kV, a distance between the nozzle and the drum of 200mm, a nozzle aperture 18G, and a spinning solution delivery rate of 15ml/h, using the methods of the present invention.
[Figure 18] Figure 18 shows the configurations of samples 1-7 used for cell culture experiments.
[Figure 19] FIG. 19 (A) shows SEM photographs (100× magnification) of sample 1 used in cell culture experiments. FIG. 19 (B) shows SEM photographs (1000× magnification) of sample 1 used in cell culture experiments. Figure 19(C) shows SEM photographs (5000× magnification) of sample 1 used in cell culture experiments.
[Figure 20] Figure 20 (A) shows SEM photographs (100× magnification) of sample 2 used in cell culture experiments. Figure 20 (B) shows SEM photographs (1000× magnification) of sample 2 used in cell culture experiments.
[Figure 21] FIG. 21 (A) shows SEM photographs (100× magnification) of sample 3 used in cell culture experiments. Figure 20 (B) shows SEM photographs (1000× magnification) of sample 3 used in cell culture experiments.
[Figure 22] Figure 22(A) shows SEM photographs (100× magnification) of sample 4 used in cell culture experiments. FIG. 22 (B) shows SEM photographs (1000× magnification) of sample 4 used in cell culture experiments.
[Figure 23] Figure 23 (A) shows SEM photographs (100× magnification) of sample 5 used in cell culture experiments. Figure 23 (B) shows SEM photographs (1000× magnification) of sample 5 used in cell culture experiments.
[Figure 24] Figure 24 (A) shows SEM photographs (100× magnification) of sample 6 used in cell culture experiments. Figure 24 (B) shows SEM photographs (1000× magnification) of sample 6 used in cell culture experiments.
[Figure 25] Figure 25(A) shows SEM photographs (100× magnification) of sample 7 used in cell culture experiments. Figure 25(B) shows SEM photographs (1000× magnification) of sample 7 used in cell culture experiments.
[Figure 26] FIG. 26 shows the results of an increase in cell number at 10 days after seeding in cell culture experiments with samples 1-7.

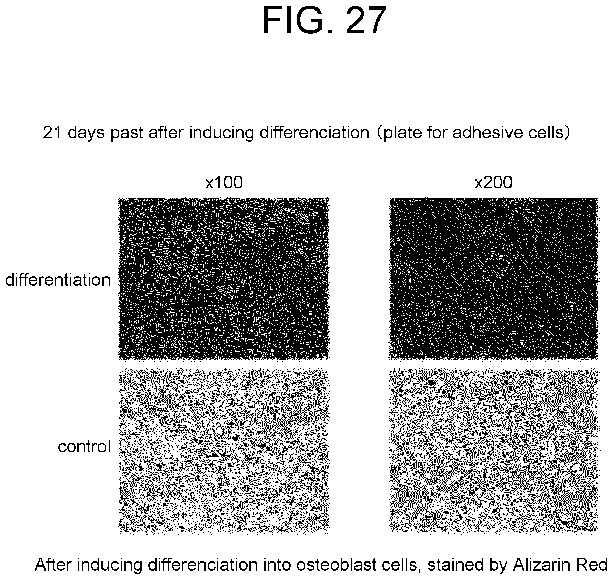

[Figure 27] FIG. 27 shows the results of a cell culture experiment using Sample 1 in which differentiation was confirmed by staining with alizaren red after 3 weeks of continuous culture using an osteoblast differentiation inducing medium 21 days after seeding.

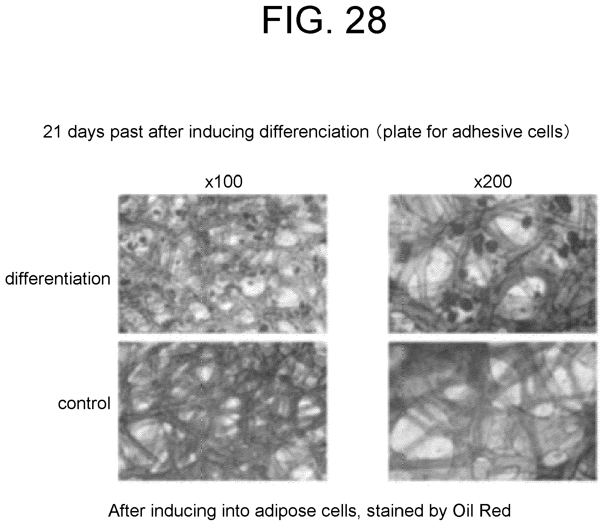

[Figure 28] FIG. 28 shows the result of confirming differentiation by staining with OIL Red after 3 weeks of continuing culture using adipocyte differentiation inducing medium after 21 days of seeding in cell culture experiment using Sample 1.

### [Embodiment of the invention]

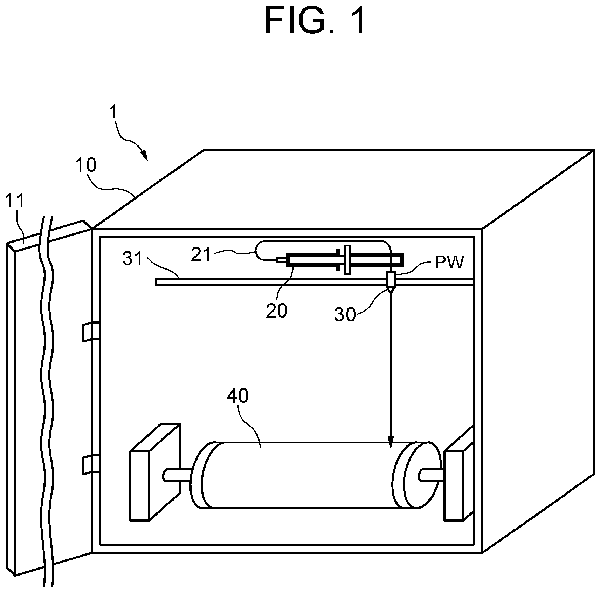

Preferred embodiments for carrying out the present invention will be described below with reference to the accompanying drawings. (1) Configuration of the electrospinning apparatus used in the present invention In FIG. 1, electrospinning apparatus 1 of the present invention includes a housing 10 that houses syringe 20, nozzle 30, and rotating drum 40. The housing 10 is preferably formed of a conductive material, such as steel, to avoid electrostatic charging. Rotating drum 40 is electrically grounded.

### <Housing >

In housing 10, it is possible to adjust temperature and humidity in the housing blocked from the outside air to some extent by closing the front door 11 which is openably attached to the front opening in accordance with individual spinning conditions. The housing 10 is provided with an exhaust fan, which can be ventilated during operation of the apparatus. Near the ceiling of the housing 10, rail 31 is mounted as a facility for sliding the nozzle 30 in the horizontal direction. In the method of the present invention, in order for the spinning solution emitted from the nozzle to be deposited on a rotary drum in a form of a fiber, the solvent must evaporate sufficiently while the ejected fiber flies in the housing 10. Therefore, it is desirable that the temperature in the housing is adjusted to 15°C to 30°C, and the humidity is adjusted to 50% or less.

### <Syringe>

Syringe 20 is fixed near the ceiling of the housing 10. Syringe 20 may be attached to a rail provided in the housing 10 and configured such that the syringe 20 itself slides along with the nozzle 30 on the rails.

After the spinning solution is filled in the syringe 20, when the switch of spinning start is pressed, spinning solution filled in the syringe 20 is extruded through the tube into the nozzle 30 at a constant pressure/speed to start electrospinning. In one embodiment of the invention, the amount of spinning solution that can be filled into the syringe is set to 10ml.

### <Nozzle>

In FIG 1, nozzle 30 is connected to syringe 20 via a tube 21. Nozzle 30 is slidably installed on a rail provided in the housing 10. Syringe 20 may be fixed to the housing 10 such that a nozzle 30 connected to the syringe 20 by a flexible tube 21 (within the reach of the tube 21) moves horizontally over the rail 31. Nozzle 30 includes a hollow needle made of a conductor, and the spinning solution extruded from the syringe 20 is introduced into the nozzle and is ejected from the tip of the needle.

### <Nozzle diameter>

The nozzle diameters used in the electrospinning apparatus of the present invention are distinguished by 27G,22G,18G according to their size. 27G,22G,18G diameters are shown in the following tables.

| Name | Outer diameter (mm) | Inner diameter (mm) |
|---|---|---|
| 27G | 0.41±0.02 | 0.22±0.03 |
| 22G | 0.72±0.02 | 0.41±0.03 |
| 18G | 1.25±0.02 | 0.82±0.03 |

### <Power supply>

A voltage adjustable DC power supply (PW) is connected to the nozzle 30. When the DC power supply is turned ON, a positive high voltage is applied to the nozzle 30. The nozzle 30 becomes a positive electrode, electrically grounded rotating drum 40 becomes a negative electrode by negatively charged to generate electrostatic induction. An electric field is formed between nozzle 30 and rotating drum 40. At the tip of the nozzle 30, a positively charged spinning solution discharged from the nozzle is subjected to electrostatic attraction, causing a Taylor cone phenomenon, which is then ejected into the air in a form of a fiber.

### <Rotating drum>

Rotary drum 40 for collecting electrospun fibers as nonwoven fabrics is installed at the bottom of the housing 10.The rotating drum 30 is electrically grounded. When a positive voltage is applied to the nozzle 30, electrostatic induction is cause and the rotating drum 40 is negatively charged to become the opposite electrode of the nozzle 30.

The rotary drum may be wound with a highly peelable sheet, such as a conductive aluminum sheet or silicone sheet. A non-woven fabric can be obtained by winding the fibers exiting and flying from the nozzle 30 around a rotating shaft of the drum. At this time, the fibers deposited on the drum are charged by the high voltage of ES, so that they repel each other on the deposited drum surface. Fast drum rotational speeds tend to orient the fibers in an ordered orientation in a winding direction, but slow drum rotational speeds dominate the repulsive forces of the fibers, resulting in a strong tendency for the fibers to orient in random directions.

### (2) Resin

The resin that can be spun by the electrospinning apparatus of the present invention is not limited to any particular kind, As far as the resin can be dissolved by using a solvent, any kind of resin can be used. As a resin, a biodegradable resin such as PLA, PLGA, PCL exhibits an adsorption performance on a hydrophobic portion of a protein because it has a hydrophobic group, and can be suitably used.

In order to fiberize the resin using an electrospinning method, in PLGA, a molecular weight of the resin is preferably 150000 to 0.4 million. More preferably, 0.2 million to 0.4 million are preferred. When the molecular weight is lower than 150000, entanglement between molecular chains becomes weak, and there is a possibility that the shape cannot be maintained as a fiber. On the contrary, if the molecular weight is larger than 0.4 million, viscosity of the spinning solution becomes too high, and it is necessary to increase the proportion of the solvent in the spinning solution to reduce the viscosity and reduce the resin concentration. In this case, however, the amount of the solvent in the spinning solution becomes too large. As a result, it becomes difficult to sufficiently volatilize the solvent during flight and it becomes difficult to fiberize the spinning solution after being emitted from the nozzle.

### (3) Volatile solvents

The solvent used in the method of the present invention is preferably a substance which dissolves a solvent-soluble resin, has high volatility at a boiling point of 200 ° C. or less under atmospheric pressure conditions, and is liquid at room temperature. Since chloroform is excellent in solubility of a biodegradable resin and is still highly volatile, it is preferable as a volatile solvent used in the method of the present invention.

### (4) Spinning solution

In the method of the present invention, resin concentration of the spinning solution needs to be kept below a certain level in order to be able to smoothly deliver the spinning solution from the syringe to the nozzle at a fast feed rate. According to experiments conducted by the inventors, when the resin concentration of the spinning solution obtained by dissolving a resin having a molecular weight of 0.2 million to 0.4 million in chloroform exceeds 8% by weight, the spinning solution is hard and difficult to be delivered smoothly.

On the other hand, it is necessary that the resin concentration of the spinning solution is equal to or higher than a certain value in order to constitute a single continuous fiber after the spinning solution emitted from the nozzle is deposited on the surface of the rotating drum and dried. According to experiments conducted by the inventors, it was not possible to form a sheet made of continuous fibers when the resin concentration of the spinning solution obtained by dissolving a PLGA resin having a molecular weight of 0.2 million to 0.4 million in chloroform is 4% by weight or less.

In the method of the embodiment of the present invention, the spinning solution does not contain an inorganic filler such as a calcium compound. When an inorganic filler is contained in the spinning solution, even under the same conditions, the fiber becomes thicker in diameter, and the fiber is broken during flight to shorten the fiber length, resulting in loss of flexibility. Furthermore, when a nonwoven fabric composed of such fibers is used as a cell culture substrate, calcium ions may be eluted with hydrolysis of the resin in a medium, and differentiation induction may work for MSCs.

### (5) Feed the syringe-filled spinning solution to the nozzle

In the method of the present invention, since the spinning solution filled in the syringe is fed at a high speed, it is considered that the diameter of the fiber emitted by Taylor-cone phenomenon increases as a result of the increase in the feed rate of the spinning solution per second at the discharge port of the nozzle. In an embodiment of the present invention, when a spinning solution filled in a syringe is delivered to a discharge port having an inner diameter of 0.4mm to 1.0mm at a rate of 3ml/h to 15ml/h, the volume feed rate of the spinning solution at the discharge port of the nozzle is from 0. 83mm³/sec to 4. 2mm³/sec, and the mass flow rate is from 1.2 mg/sec to 6.8 mg/sec.

### (6) Applying voltage to the nozzle

Fill the syringe with the spinning solution, turn on the DC power supply, and apply a voltage to the nozzle 30. By applying a voltage to the nozzle 30, the filled spinning solution is charged and a potential difference is created between the installed drum collector and the nozzle, and the charged spinning solution is pulled in a direction toward the drum collector by the Taylor-Cone phenomenon.

In the method of the present invention, the fibrous spinning solution emitted from the nozzle is not rapidly narrowed nor nano-fiberized by the force of swinging the flight trajectory in the electric field, but is deposited on the drum having a micrometer range fiber diameter. Although the reason is not necessarily clear, according to the inventors' observation, it is believed that when the spinning solution ejected from the nozzle falls down under the influence of electric force and gravitational force, ultra-fibrillation of spinning solution that is caused by bending instability phenomena in typical electrospinning does not occur even when the emitted spinning solution is subjected to the repulsive force caused by the bias of electric charge. In the method of the present invention, decrease of fiber diameter seems to occur simply due to evaporation of the solvent during flight and swinging of flight trajectory of the fibers.

In order for causing swinging phenomenon of the flight trajectory of the present invention to occur, it is necessary that the electric field formed between the nozzle and the rotating drum is equal to or greater than a certain degree. Since the strength of the electric field is determined by the value of the applied voltage and the distance between the nozzle 30 and the rotating drum 40 based on the formula V = Ed, the value of the applied voltage required to cause the swinging phenomenon of the flight trajectory is not determined independently. However, since the flight distance of the fibers emitted from the nozzle needs to be greater than a certain level since the solvent must evaporate therebetween, the value of the applied voltage is necessarily set high. In one embodiment of the present invention, the distance between the nozzle and the drum is 200mm, and the voltage applied to the nozzle is set to 28kV.

In the fibers spun by the method of present invention, numerous bubble pores having a diameter of about 1µm are extensively formed over the entire area of the fiber surface. The mechanism by which such bubble pores are formed is that the fibers emitted from the nozzle contain a large amount of chloroform, and the chloroform contained in the fibers becomes bubbles inside the fiber and on the surface of the fibers during flight and evaporates. When air bubbles exit the fiber from inside, several air bubbles combine to form larger air bubbles so that surface area of the bubbles is decreased and exit the fiber. It is believed that the bubbles generated near the surface of the fiber will be smaller than the bubbles generated deep inside the fiber. Fibers emitted from the nozzle are constantly exposed to fresh air by flying through the air and continue to receive thermal energy, thereby facilitating vaporization near the surface of the fiber. A large number of bubbles may be generated by receiving a large amount of energy, and some may be combined to form larger bubbles in order to reduce the surface area of the bubbles.

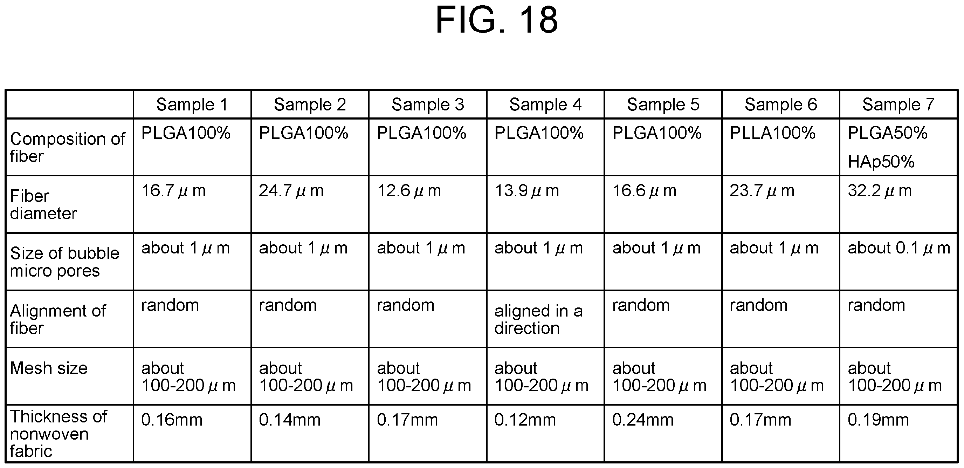

Size of the bubble pores formed on the surface of the resin fibers is affected by the viscosity of the polymer. In the embodiment of the present invention, diameter of the bubble pores formed on the surface of the fibers was 0.1 to 3µm (see FIG. 18). While size of the bubble pores formed on the surface of the fibers spun by electrospinning is typically several nm or less, the bubble pores formed on the fiber surface of the present invention are considerably larger than those. The bubble pores formed in the resin fibers spun by the method of the present invention are large enough for the cells to sense the pores as physical irregularities on the polymer surface. And the pores are considerably larger than the size of the protein molecules (BSA is 8.5 nm, and lysotium is 3.8 nm). Therefore, it is possible for the proteins contained in the medium to enter and be accommodated in the bubble pores in the fibers.

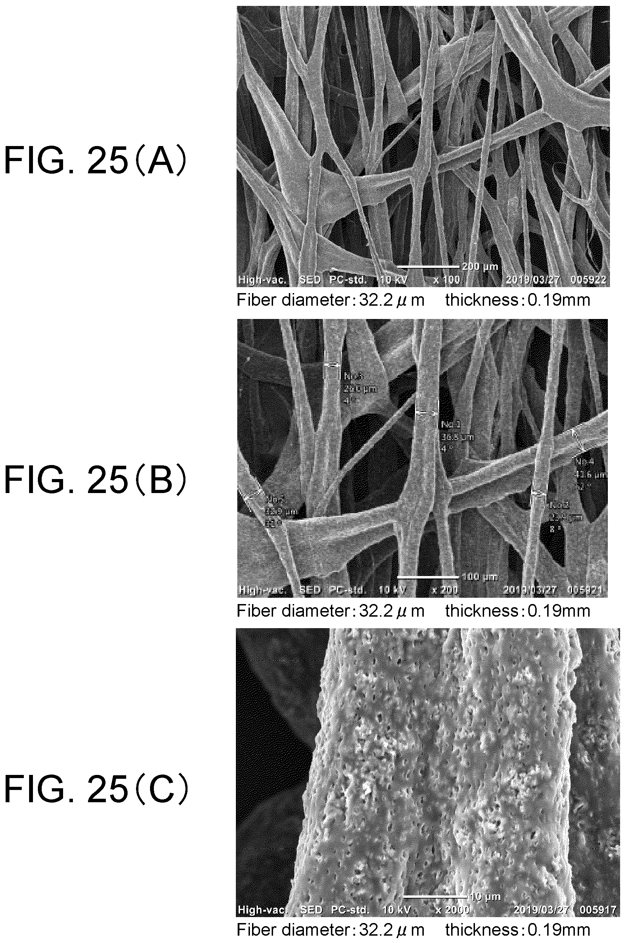

In order to form bubble pores having a diameter of about 1µm on the surface of a resin fiber to be spun by electrospinning, it is desirable that the spinning solution is made of a resin dissolved in a solvent and does not contain an inorganic filler. When inorganic particles are contained in the spinning solution, formation of bubbles is prevented in the fibers emitted from the nozzle, so that bubble pores are hardly formed on the fiber surface. In our experience, when the inorganic particles are more than 30% by weight, the bubble pores formed on the fiber surface become significantly smaller and the diameter of the pores becomes smaller. In Comparative Sample 7 shown in FIG. 25 (C), bubble pores having a diameter much less than 0.1µm were scattered on the surface of the fibers spun at a composition ratio of PLGA50 wt%, HAp particles 50 wt%.

### (7) Collect non-woven fabrics

The nonwoven fabric sheet can be collected by depositing fibers as nonwoven fabrics on a rotary drum wound with a highly peelable sheet, such as an aluminum sheet or silicone sheet, and then removing the aluminum sheet from the rotary drum. In the method of the present invention, since the length of the spun single fibers is 20cm or more and reaches the drum in a wound state by swinging the trajectory during a falling down flight, a plurality of long fibers which are wound and become curved are entangled with each other on the drum to constitute a nonwoven fabric.

### I. Nonwoven fabric production experiments

Experiments 1, 2, 3, and 4 were carried out to fabricate nonwoven fabrics consisting of micrometer range diameter fibers spun by an electrospinning method using spinning solutions prepared by dissolving PLGA resins in chloroform.

### [Experiment 1]

PLGA having a molecular weight of 340000 (LG855S produced by Evonik Ind.)was dissolved in chloroform having a purity of 99% or more to prepare a spinning solution having a resin concentration of 6% by weight. The prepared spinning solution was used to spin by using an electrospinning method.

### 1) ES condition

ES Equipment: NANON-03 (provided by MECC Co., Ltd)
   Solvent: chloroform purity 99% or more
   Resin concentration in solvent: 6 wt%
   Voltage :28 kV
   Extrusion rate: 10 ml/h
   Needle Thickness: 18G
   Distance from nozzle to collector: 200 mm
   Collector: Rotating drum (rotation speed 50rpm).

### 2) Results of Experiment 1

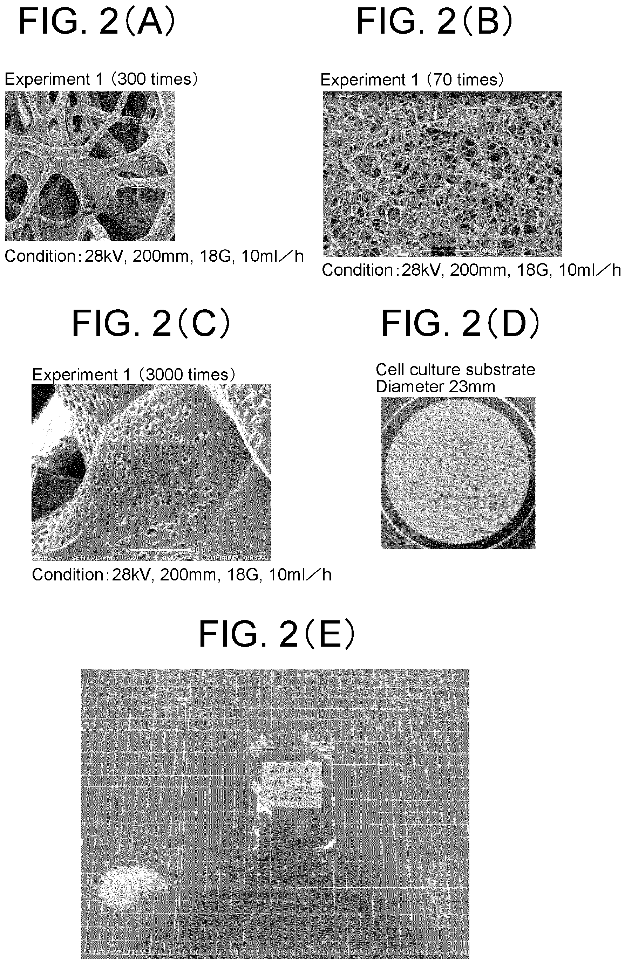

- The fibers emitted from the nozzle fell down in the falling direction while swinging the flight trajectory, and it was observed that the fibers were deposited on the rotating drum.
- Fiber diameters of the fibers deposited on the drum were approximately 10-20µm.
- Fibers deposited on the rotating drum formed a sheet with a network structure in which a plurality of fibers are entangled each other (see Figures 2 (A), (B), and (C)).
- In order to measure the length of the fibers spun in Experiment 1, the fibers were injected into a container filled with ethanol liquid instead of a rotating drum as a collector, and the collected cotton was recovered into a cotton shape, and the length of the fibers constituting the cotton was measured to be about 50cm (see Figure 2 (E))

### [Experiment 2]

### 1) ES condition

Under the same conditions as in Experiment 1, spinning solution was prepared and spun using the same electrospinning apparatus. Provided however that, voltage was 21kV, flying distance from the nozzle to the rotating drum was 150mm

### 2) Results of Experiment 2

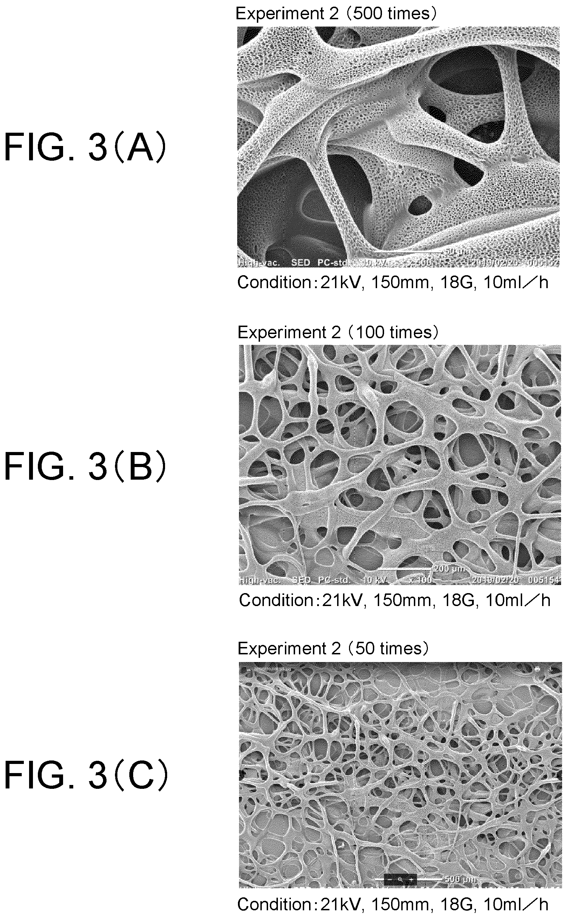

- The fibers emitted from the nozzle fell down in the falling direction while swinging the flight trajectory and were deposited on the rotating drum. The flight trajectory of the fiber swung before the fibers were deposited on the drum, but the degree of swinging was less than that in Experiment 1.
- Diameters of the fibers deposited on the drums were not necessarily constant in the longitudinal direction, and there were thin and thick portions, but were generally in a range of 10-50µm.
- The fibers deposited on the rotating drum formed a network structure, but had a stronger tendency to adhere each other between the fibers than in Experiment 1 (see FIG. 3).

### [Experiment 3]

### 1) ES condition

Under the same conditions as in Experiment 1, spinning solution was prepared and spun using the same electrospinning apparatus. Provided however that, the voltage was 14kV, flying distance from the nozzle to the collector was 100mm.

### 2) Results of Experiment 3

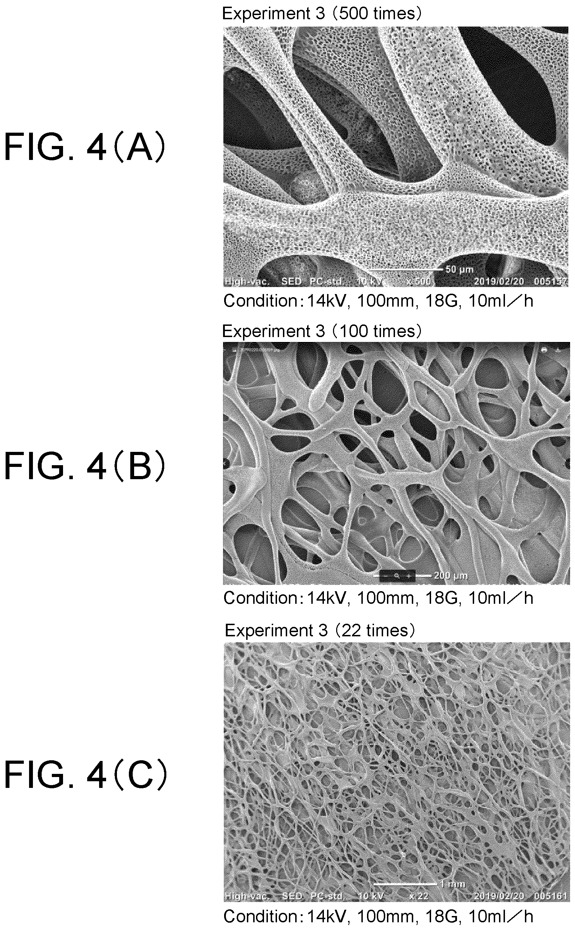

- The fibers emitted from the nozzle fell down in the falling direction while swinging the flight trajectory and were deposited on the rotating drum.
- Fibers deposited on the drum had a fiber diameter of 10 to 50µm and a fiber of several µm to nanometers.
- Fibers deposited on the rotary drum formed a network structure, but diameter of the fibers varied more than in Experiment 2, and the tendency to adhere each other between the fibers was stronger (see Fig. 4).

### [Experiment 4]

### 1) ES condition

Under the same conditions as in Experiment 1, spinning solutions was prepared and spun using the same electrospinning apparatus. Provided however that, diameter of the nozzle was 22G.

### 2) Results of Experiment 4

- Fibers emitted from the nozzle fluctuated sharply in the middle of the flight trajectory, making it invisible.
- Fibers deposited on the drum had a fiber diameter of 5µm to 60µm, which was adhered to each other at the point where the fibers contacted each other, forming a network structure.

### «Evaluation of Experiments 1, 2, 3, and 4»

### i) Distance between nozzle and drum

In Experiments 1, 2, 3 and 4, the distance between the nozzle and drum is 150mm in Experiment 2 and 100mm in Experiment 3, which is shorter than Experiment 1, but the strength of the electric field calculated by the formula E=V/d is the same for all four. From the comparison of the results of Experiments 1, 2, and 3, when the distance between the nozzle and the drum is longer (200mm), the fiber deposited on the drum maintained the shape of the fiber having a certain range of diameter and formed a sheet with a network structure, whereas when the distance between the nozzle and the drum is shorter (150mm in Experiment 2 and 100mm in Experiment 3), diameter of the fiber deposited on the drum was not constant, and the fibers tended to adhere to each other. From this, it is considered that in order to form a sheet made of fibers of several tens µ m, existence of a distance sufficient to evaporate the solvent when the fibers emitted from the nozzle are deposited on the drum is important.

### ii) Nozzle diameter

Comparing the fibers and the nonwoven fabric prepared in Experiment 1 and Experiment 4, fibers of several 10 of µ m were uniformly spun in Experiment 1 using a nozzle having a 18G caliber, and fibers having a large diameter adhered to each other to form a nonwoven fabric in Experiment 4 using a nozzle having a 22G caliber, At a feed rate of 10 ml/h, it is considered that 18G is superior to 22G as a spinning condition for fibers.

### Comparative Experiment

Comparative experiments 1, 2, 3, and 4 in which the conditions of Experiment 1 described above were changed were performed to find out the conditions necessary for forming a sheet made of microfibers.

### [Comparative Experiment 1]

### 1) ES condition

Under the same conditions as in Experiment 1, spinning solutions were prepared and spun using the same electrospinning apparatus. Provided however that, 27G was used for the nozzle, and feed rate of the spinning solution was set to 1ml/h.

### 2) Results of comparative experiment 1

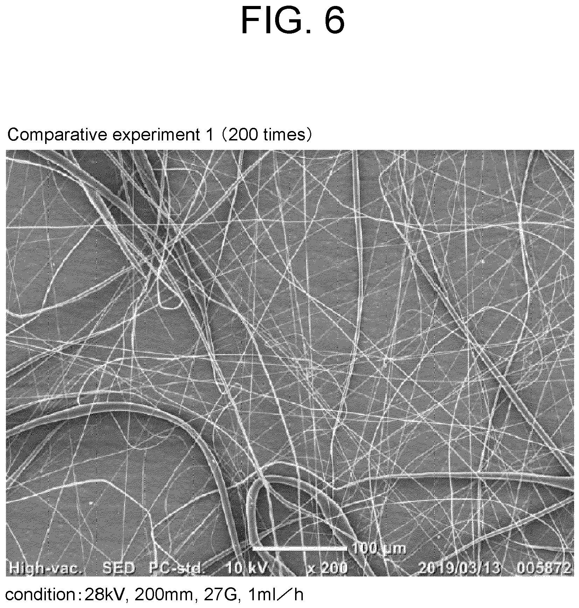

- Fibers emitted from the nozzle soon became invisible of the flight orbit.
- Fiber diameters deposited on the drum were mixed with those of 500nm to 800nm and those of several µm in diameter (see Fig. 6).

### [Comparative Experiment 2]

### 1) ES condition

Under the same conditions as in Experiment 1, spinning solutions were prepared and spun using the same electrospinning apparatus. Provided however that, diameter of the nozzle 27G was used.

### 2) Results of comparative experiment 2

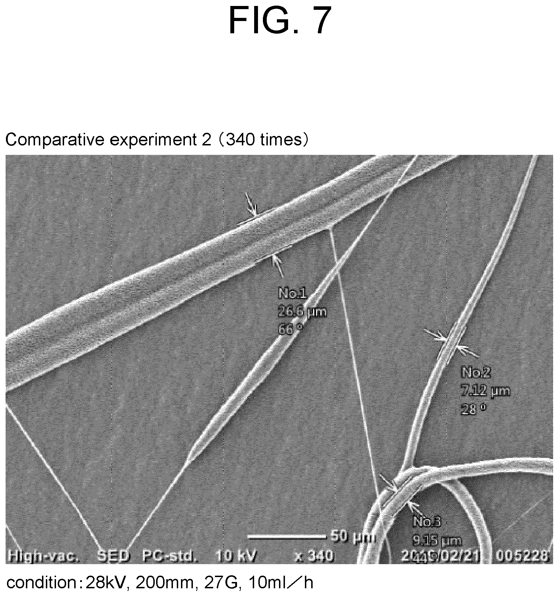

- The fiber emitted from the nozzle disappeared because the flight trajectory swang violently on the way.
- The syringe has been overloaded and the fixation has failed. The reason for this is considered to be that pressure of the solution that could not pass through the diameter went to the syringe fixture due to the fast feed rate.
- Diameter of the fibers deposited on the drum was thin. It was a mixture of the fibers having diameter of 0.5nm to several µm (see Fig. 7).

### <Evaluation of Comparative Experiment 2>

At a syringe-to-nozzle feed rate of 10ml/h and 27G nozzle bore size, stable spinning could not be achieved, and nonwoven fabrics were not formed. At a feed rate of 10ml/h, nozzle diameter18G is considered to be suited.

### [Comparative Experiment 3]

### 1) ES condition

Under the same conditions as in Experiment 1, spinning solutions were prepared and spun using the same electrospinning apparatus. Provided however that, the resin concentration was 4% by weight, and the distance between the nozzle and the drum was 300mm.

### 2) Results of comparative experiment 3

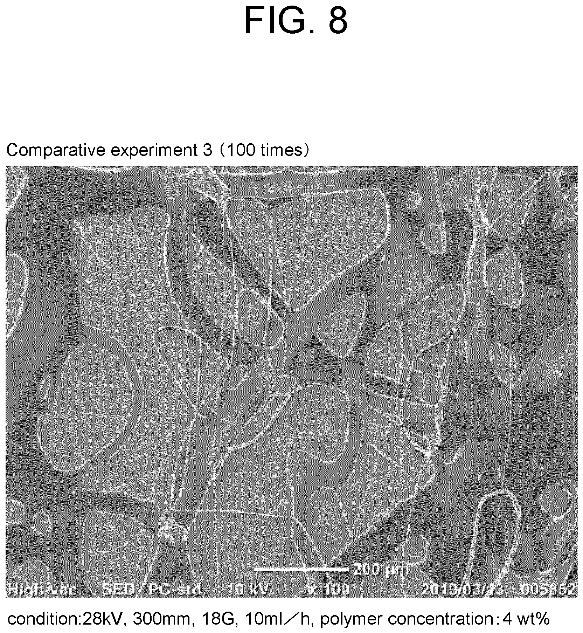

- Fibers emitted from the nozzle fell straight downward without swinging the flight trajectory and accumulated on the drum.
- Nanofibers and thick fibrous ones were mixed and deposited on the drum, and the length of the fibers was short and did not become a nonwoven fabric (see FIG. 8).

### <Evaluation of Comparative Experiment 3>

Since the amount of the solvent of the spinning solution was large and the amount of the resin was small at the resin concentration of 4% by weight, the spinning solution emitted from the nozzle could not form a single continuous fiber. It is considered that the solvent does not evaporate enough from the fibers during flight and falls in a state in which an excessive amount of solvent relative to the amount of the resin remains, so that it is deposited as it is on the drum. As a result, a fiber containing an excessive amount of the solvent collides with the surface of the drum and adheres thereto.

### Experiments (applied voltage and flight trajectory)

Under the same conditions as in Experiment 1, provided however that, a voltage applied to the nozzle was 0.5 k V,10kV,14kV and 18 k V,22kV,25kV,28kV,30kV and changes of the flight trajectory of emitted fiber and the conditions of the fibers deposited on the rotating drum were observed.

### Experimental results:

i) Changes of flight trajectory
   - 0.5 At kV, the spinning solution is pushed out of the nozzle and fell down.
   - At 10 kV, the spinning solution fell downward in a form of short filaments.
   - At 14 kV, the spinning solution fell onto the drum in a single line. A small swinging of flight trajectory was observed.
   - At 18kV, the fibers emitted from the nozzle were deposited on the rotating drum while the flight trajectory swung greatly.
   - At 22 k V,25kV,28kV,30kV, the fibers emitted from the nozzle were pulled with stronger force and deposited on the rotating drum. Before accumulating on the rotating drum, the flight trajectory was deflected and the fibers became invisible to naked eye.
ii) State of the fibers deposited on the rotating drum
   - When the applied voltage was 14kV or less, the fibers deposited on the rotating drum did not form a nonwoven fabric.
   - When the applied voltage was 18kV to 30kV, the fibers deposited on the rotary drum formed a nonwoven fabric. However, when the applied voltage was 30kV, the fiber tended to adhere strongly to each other more than at 18kV to 28kV and the shape of the fibers collapsed.

### Experiments (feed rate of spinning solution and nonwoven fabric formation)

By changing the rate at which the spinning solution was delivered from the syringe to the nozzle from 3ml/h to 15ml/h, effect to the formation of nonwoven fabrics was observed

### Experimental results

- Fibers deposited on the rotating drum at a feed rate of 3ml/h to 15ml/h formed a mesh-structured sheet. However, at a feed rate of 12ml/h or more, the tendency of adhesion between fibers was strong, and the shape of the fibers collapsed, and it tended to adhere flat to the drum.

### II. MSC proliferation experiment

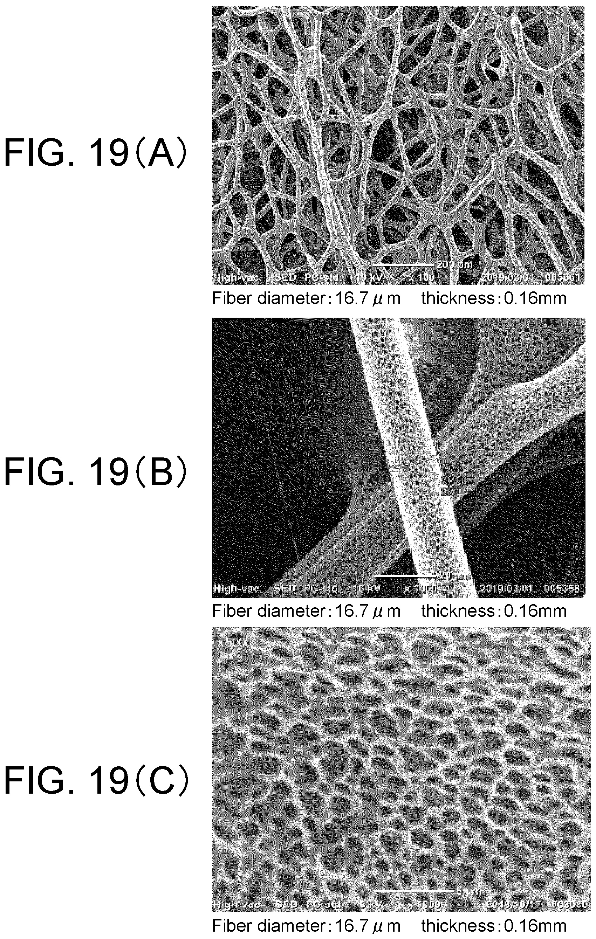

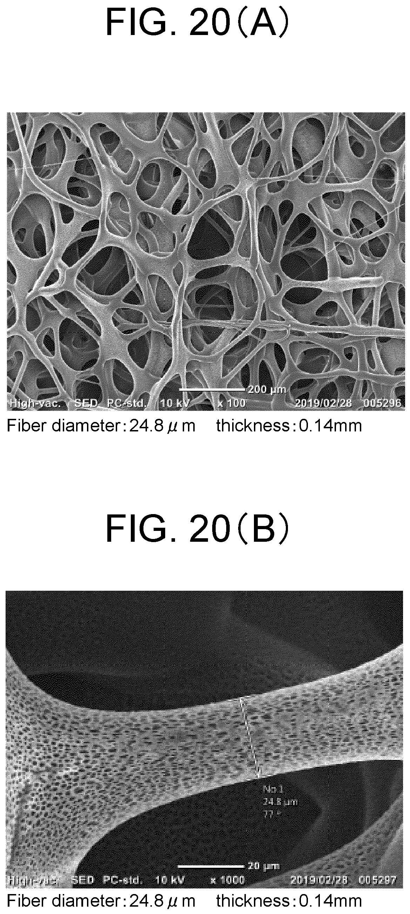

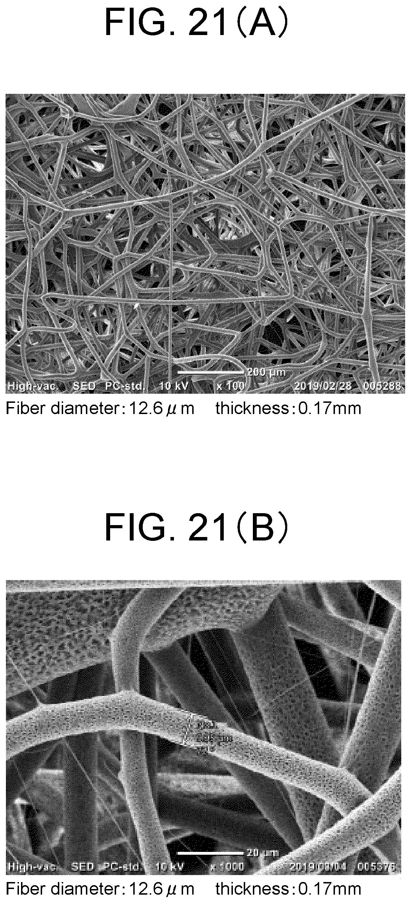

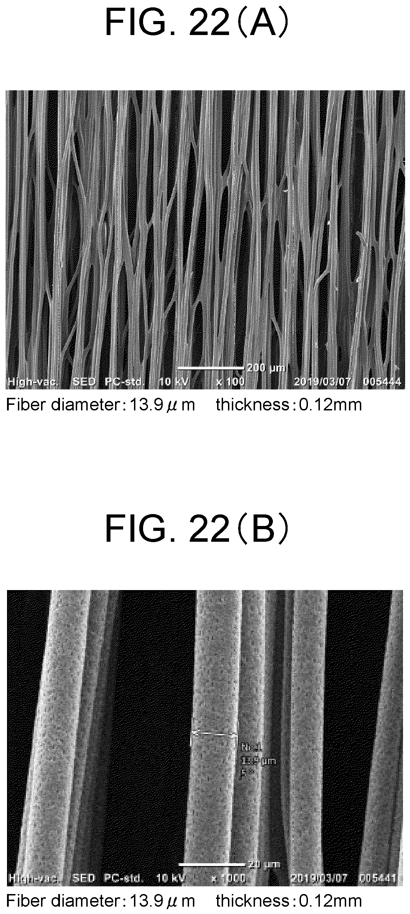

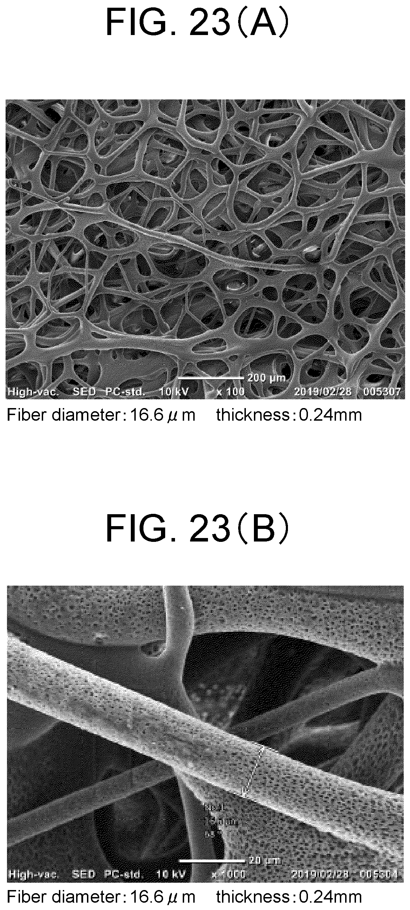

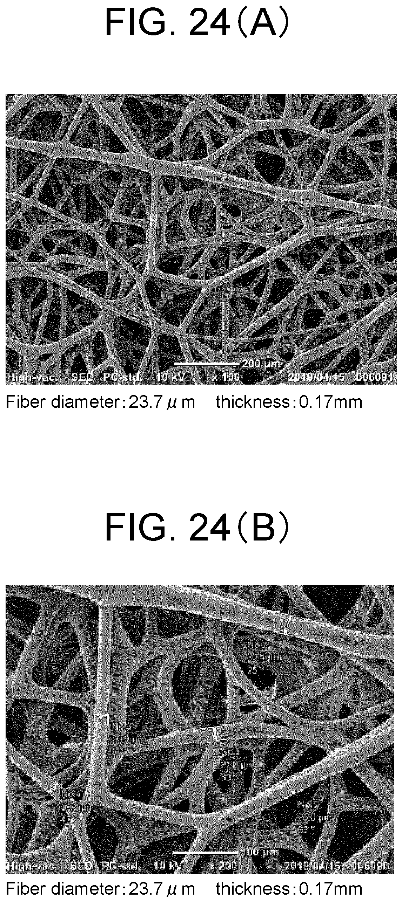

Nonwoven fabric sample 1-6 made of resin fibers produced by an electrospinning method and comparison sample 7 were prepared. By seeding MSCs, proliferation of cells in each sample was observed and compared. FIG. 18 shows the configuration of samples 1-6 and comparison sample 7, and FIGS. 19-25 show SEM photographs of each sample.

### 1) Experimental procedure

Step 1: Adipose-derived mesenchymal stem cells (AT-MSC) were suspended in serum-free culture medium (culture medium at the time of cryopreservation) at the bottom of the flask to achieve 1 x 10⁴ cells/cm² cell density, and the resultant suspension was seeded in a T25 flask (Falcon #353109) by adding 5mL of culture medium (25 x 10⁴cell/flask). Cells inoculated into T25 flasks were pre-incubated at 37°C and 5%CO₂, and the entire culture medium was replaced on 3^{rd} day of incubation and the preincubation was continued for 5 days.

Step 2: After completion of the pre-culture, the culture solution for cryopreservation was removed from the cells, washed with PBS(-) (strain) Cell Science Laboratory, #1102P05), and then the cells proliferated in the pre-culture were detached from the bottom surface of the T25 flask using recombinant trypsin/EDTA solution (r-TE (strain) Cell Science Laboratory, #1210)), and then the cells were harvested by PBS(-) (strain) Cell Science Laboratory, #1102P05) after adding 0.5 mL/ flask of synthetic trypsin neutralizing solution (s-TI (strain) Cell Science Laboratory, #1220) and allowing them to fit, and then transferred to a 15-mL centrifuge tube.

Step 3: Cells transferred to a centrifuge tube were centrifuged (5 min at 1000 rpm), and the separated cells were suspended in serum-free culture (CiMS) for proliferation. As a culture medium for proliferation, CiMS-BM ® (#A2G00P05C of the Institute of Cell Science, Inc.) was added with CiMS-sAF (#A2G20P1CC of the Institute of Cell Science, Inc.) and used.

Each sheet of Samples 1-7 was fixed to the bottom of each 24-well adhesion plate (Falcon #353046, 351147) and the cells were seeded on the sample sheets 1-7 in that condition at a seeding density of 5 x 10⁴ cells per sheet. Cultures were started at 37°C and 5%CO₂, and the media were replaced every 3 days.

### 2) Experimental results

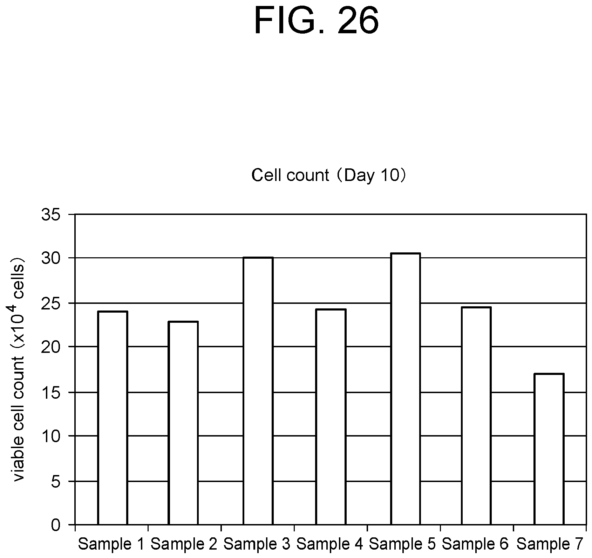

- Increase of cell numbers after 10 days of seeding of AT-MSC suspensions in samples 1-7 is shown in Fig. 26.
- Cell proliferation in Sample 3 and Sample 5 was greater than that in the other samples at the 10-day time point.
- Viability of the cells recovered on day 10 was around 90% in all samples.
- After 10 days, cultures were continued every 3 days. Although accurate cell count was difficult, proliferation of cells was observed after 20 days. Cells ≧ 60x10⁴cells/sheet were counted at 30 days and the growth rate was still not plateaued at that time.

### 3) Discussion

i) Samples 1-6 showed good cell proliferation after 10 days of seeding. The reason for this is considered to be that proteins contained in the medium (usually about 2-10nm in size of protein) entered into the pores due to the myriad formation of bubble pores having a size of about 1µm on the surface, and the initial adhesion was improved by being effectively adsorbed.
ii) It is considered that the reason why the cell proliferation was larger in Sample 3 and Sample 5 than in the other samples at the time of 10 days passed is that Sample 3 has a smaller fiber diameter (12.6µm) and Sample 5 has a larger thickness of the nonwoven fabric (0.24mm), so that both of them have a larger surface area of the entire nonwoven fabric, so that the cells can adhere more.
iii) Despite that fiber diameter of sample 6 (PLLA100%) is 23.7µ thick, high proliferation next to the proliferation in samples 3 and 5 was achieved. And MSCs adhered to the nonwoven fabric was easily detached from the nonwoven fabric by using trypsin. It is considered that PLLA has an excellent property as a resin material of cell culture substrate.
iv) Comparison sample 7 (containing 50 wt.% HAp-particles relative to PLGA) had lower cell growth numbers than samples 1-6. The reason for this may be attributed to the facts that the surface of the fibers of the HAp comparison sample 7 (Figure 25(C)) has smaller number of pores and thus flatter than that of the fiber surface of Sample 1-6, and the surface of the fiber of sample 7 has less unevenness. Also, because the fiber of sample 7 is thick, it is difficult to capture the cells in the nonwoven fabric. And further, specific surface area of sample 7 is considerably smaller than that of samples 1-6.
v) Cell proliferation was observed in samples 1-6 after 20 days of inoculation, and the plateau had not yet been reached at 30 days, so further growth is expected after 30 days. Although it is not clear why such a result was obtained, according to the knowledge and experience of the inventors, the adhesion area of the cells was increased by the nonwoven fabric, and the mesenchymal stem cells seeded on the nonwoven fabric were effectively captured, and the cells adhered to the fiber surface covered with the protein.
vi) The reason why the number of cells grown in the sample 4 was smaller than that in the samples 3 and 5 is considered to be that the seeded AT-MSC often passed through the nonwoven fabric in the medium because the fibers in the sample 4 aligned in one direction and did not form a mesh structure.

### III. Experiments to confirm differentiation potential of proliferated MSCs

### 1) Experimental procedure

In the MSC proliferation experiment of the above II., AT-MSC were seeded on a sheet of Sample 1 described above, and culture was continued for 20 days while changing the culture solution for serum-free proliferation (CiMS) every 3 days. To induce differentiation thereafter, the culture medium for proliferation was removed and washed with PBS. Then, the culture medium for inducing serum-free differentiation was added, and the culture was started at 37 °C and 5%CO2, and the culture was continued while the culture medium was changed every 3 days, and the differentiation was confirmed by staining after 3 weeks elapsed.

IMDM, 10% FBS, 0.1 µM Dexamethasosne, 0.2mM Ascorbic acid, 10 mM β-Glycerol-2-phosphate, 50ng/mL hEGF of compositions were used as culture medium for induction of serum-free bone differentiation. Compositions of IMDM, 10% FBS, 0.1 µM Dexamethasosne, 0.5mM IBMX, 2µg/mL Inslin, and 0.1mM indomethacine were used as culture medium for inducing lipid differentiation.

Without induction of differentiation, culture medium for proliferation was used as a control and maintained for 3 weeks in the same period as the induction of differentiation. Comparison was observed by staining.

### 2) Experimental results

After differentiation induction was carried out by culturing for 21 days using a culture medium for inducing osteogenesis, the cells adhered to the cell culture substrate were stained with Alizarin Red, and it was observed that the cells were stained and differentiated into osteoblasts. After differentiation induction was performed by culturing for 21 days using a culture medium for inducing adipogenic differentiation for 21 days, the cells adhered to the cell culture substrate were stained with Oil Red, and it was observed that the cells were stained and differentiated into adipocytes. The control was not stained in either Alizarin Red or Oil Red.

### 3) Discussion

Since all cells adhered to the cell culture sheet of Sample 1 were found to differentiate into both cells by staining after 21 days of induction of differentiation into osteoblasts and adipocytes, it is considered that MSC cells seeded on the nonwoven sheet proliferated in the proliferation medium while maintaining their differentiation potential. Because none of the controls were stained, MSCs proliferation in proliferation medium were considered to have proliferated without being differentiated.

While the present invention has been described in accordance with embodiments of a nonwoven fabric sheet made of fibers spun using an electrospinning method, the sheet of the present invention is not limited to a nonwoven fabric as long as it can be used as a cell culture substrate, and is included in the scope of the present invention as long as it has a structure in which cells can enter and adhere.

### [Explanation of notations]

1 Electrospinning apparatus
10 Housing
11 Front door
20 Syringe
21 Tube
30 Nozzle
31 Rail
40 Rotating drum

## Claims

1. A cell culture substrate made of a nonwoven fabric produced by using an electrospinning method,
the nonwoven fabric comprises a plurality of resin fibers having an outer diameter of 10 to 50µm, wherein the plurality of resin fibers entangle each other in random directions,
the plurality of resin fibers adhere and connect with each other at a plurality of locations to constitute a network structure, the network structure comprises substantially oval-shaped mesh holes having a diameter of 100-200µm surrounded by curved resin fibers,
the resin fibers constituting the nonwoven fabric do not contain an inorganic filler, and the resin fibers have numerous bubble pores with a diameter of 0.1 to 3µm formed over an entire surface of the resin fibers.

2. The cell culture substrate of claim 1, wherein a thickness of the nonwoven fabric is 0.1mm to 0.4mm.

3. The cell culture substrate according to claim 1 or 2, wherein the resin fiber comprises a PLGA resin having a molecular weight of 0.2 million to 0.4 million.

4. A cell culture substrate according to any one of claims 1 to 3, wherein the resin fibers constituting the nonwoven fabric do not contain an inorganic filler.

5. A method for producing a cell culture substrate made of a nonwoven fabric produced by using an electrospinning method, comprising:
filling a syringe of an electrospinning apparatus with a spinning solution having a resin concentration of 5wt% to 7wt% prepared by dissolving a biodegradable resin having a molecular weight of 50000 to 0.7 million in chloroform;
delivering the spinning solution to a nozzle of the electrospinning apparatus having an inner diameter of 0.7mm - 0.9 mm so that the spinning solution is fed to a discharge port of the nozzle at a feed rate of 3ml/h to 15ml/h;
applying a predetermined voltage to the nozzle to generate an electric field between the nozzle and a drum collector which is electrically grounded and installed at a distance of 150mm to 400mm from the nozzle so that electrically charged spinning solution delivered to the nozzle is emitted from the discharge port of the nozzle in a form of a resin fiber by Taylor-cone phenomenon, wherein strength of the electric field is adjusted such that flight trajectory of the resin fibers emitted from the nozzle is swung during flight to the drum collector;
depositing the resin fibers emitted from the nozzle on a surface of the drum collector that is rotating at a predetermined speed so that curved resin fibers are aligned in random directions and adhered with each other at a plurality of locations to form a nonwoven fabric on the drum collector, wherein the resin fibers having a diameter of 10µm to 100µm form a network structure comprising substantially oval-shaped mesh holes having a diameter of 100-200µm surrounded by the curved resin fibers; and
collecting the nonwoven fabric from the drum collector and cutting to a desired size.

6. The method for producing a cell culture substrate according to claim 5, wherein a thickness of the nonwoven fabric is 0.1mm to 0.4mm.

7. A process for producing a cell culture substrate according to claim 5 or 6, wherein said resin fiber comprises a PLGA resin having a molecular weight of 0.2 million to 0.4 million.

8. The method for producing a cell culture substrate according to any one of claims 5 to 7, wherein the resin fibers constituting the nonwoven fabric do not contain an inorganic filler.
